# EUROPEAN PATENT APPLICATION

(11) **EP 4 692 342 A1**
(43) Date of publication of application: **11.02.2026**
(21) Application number: 24779699.8
(22) Date of filing: 19.03.2024
(51) Int. Cl.: C12N 15/10, C08F 220/36, C12N 9/12, C12N 15/12, C12Q 1/6844

(54) **SINGLE-STRANDED NUCLEIC ACID MODIFIER, AQUEOUS NUCLEIC ACID SOLUTION, AND NUCLEIC ACID AMPLIFICATION METHOD**

(30) Priority: 30.03.2023 JP 2023055218
(71) Applicant: NOF Corporation, Shibuya-ku Tokyo 150-6019 (JP)
(72) Inventor: SUZUKI, Hirotaka, Kawasaki-shi, Kanagawa 210-0865 (JP); MATSUDA, Masaru, Kawasaki-shi, Kanagawa 210-0865 (JP)
(74) Representative: J A Kemp LLP
(86) International application number: PCT/JP2024/010664
(87) International publication number: WO 2024/203574

(57) **Abstract**

The present invention provides a single-stranded nucleic acid denaturant consisting of a copolymer having constitutional units (a) derived from a monomer having a phosphorylcholine group and constitutional units (b) derived from a monomer having a carboxy group, wherein the content of constitutional units (b) relative to the total constitutional units of the copolymer is 20 to 90 mol%.

## Description

### [Technical Field]

The present invention relates to a single-stranded nucleic acid denaturant, a nucleic acid aqueous solution and a nucleic acid amplification method.

### [Background Art]

RNA (Ribonucleic acid) is a biopolymer in which monomeric ribonucleotides are linked by phosphodiester bonds.

RNA naturally consists of ribonucleotides containing the four nucleic acid bases (adenine, guanine, cytosine, and uracil), and derivatives thereof. In the body, it has functions such as transmitting genetic information, signal transduction, transporting amino acids in protein synthesis, metabolic control, constituting ribozymes, and constituting organelles. It also exists as RNA with unknown functions, and constitutes genomes of some viruses.

Examples of uses of RNA in society include therapeutic targets in the medical field, components of nucleic acid drugs, markers for detecting viruses and microorganisms in infectious disease treatment and environmental surveys, and components of mRNA vaccines. Another example of use is as a component of genome editing tools in genetic engineering, such as guide RNA for site-specific nucleases such as CRISPR/Cas9 (Clustered regularly interspaced short palindromic repeats/crispr associated protein 9). Yet another example is use as a raw material for health foods and food additives.

In basic research, applied research, and other molecular biology research in the above-mentioned fields, RNA is often separated by length. The purpose thereof varies, including isolating specific RNAs, identifying RNA species, understanding the state of RNA contamination in biological samples, base sequencing, and detecting specific sequences by Northern blotting.

Electrophoresis method is a widely used as a method for separating RNA by length. Examples of the aforementioned method include agarose gel electrophoresis method, polyacrylamide gel electrophoresis method, and capillary electrophoresis method, which applies these methods.

Natural RNA is generally synthesized as a single strand, but it is known to form higher-order structures by forming complementary base pairs (adenine and uracil, guanine and cytosine) within the molecule or by forming non-specific hydrogen bonds. This can cause problems when separating RNA by length. To be specific, the principle of separation by electrophoresis method is molecular sieving: when RNA forms a higher-order structure within the molecule, its apparent size becomes smaller than that of linear RNA of the same length, and its migration distance becomes longer. Therefore, in order to separate RNA by length, it is necessary to loosen the higher-order structure of the RNA to linearize same, i.e., to denature the RNA. Similar problems also exist with single-stranded DNA.

In electrophoresis method, as a method for denaturing RNA or single-stranded DNA, it is widely performed to add a denaturant to a sample containing RNA to be separated, or to a gel or buffer used for electrophoresis. Formaldehyde is often used as such denaturant (Patent Literature 1). However, formaldehyde is difficult to handle due to its extremely high volatility. Furthermore, formaldehyde is toxic and irritating, and is suspected of being carcinogenic and mutagenic.

Formamide and urea are sometimes used as denaturants (Patent Literature 2). Formamide and urea improve the problems of formaldehyde in terms of volatility and safety.

### [Citation List]

### [Patent Literature]

[Patent Literature 1]
   JP 2002-521071 A
[Patent Literature 2]
   JP 2023-512829 A

### [Summary of Invention]

### [Technical Problem]

Conventionally used formamide and urea are less effective as denaturants. Furthermore, formamide generally needs to be added also to the electrophoresis buffer or gel. Therefore, when using formamide, buffers and gels different from those required for standard electrophoresis method (electrophoresis method under non-denaturing conditions) must be prepared, which is inconvenient. In addition, when using formamide, the electrophoresis sample must be boiled immediately before performing an electrophoresis method, which is also time-consuming and inconvenient.

Furthermore, the denaturant polymethacrylic acid inhibits enzyme reactions. Therefore, a nucleic acid aqueous solution containing polymethacrylic acid and single-stranded nucleic acid cannot be subjected to enzyme reactions.

The present invention has been made in consideration of the above-mentioned problems, and aims to provide a single-stranded nucleic acid denaturant that is low volatile, easy to handle, highly safe, expresses a denaturing effect on single-stranded nucleic acids even by an electrophoresis method under non-denaturing conditions, does not require boiling of samples before electrophoresis, and does not inhibit enzyme reactions.

### [Solution to Problem]

The present inventors have conducted intensive studies and found that the above-mentioned object can be achieved by using the following copolymer as a single-stranded nucleic acid denaturant. Based on this finding, the present invention provides the following.
[1] A single-stranded nucleic acid denaturant consisting of a copolymer containing constitutional units (a) derived from a monomer having a phosphorylcholine group and constitutional units (b) derived from a monomer having a carboxy group, wherein the content of constitutional units (b) relative to the total constitutional units of the copolymer is 20 to 90 mol%.
[2] The single-stranded nucleic acid denaturant of the aforementioned [1], wherein the monomer having a phosphorylcholine group is (meth)acryloyloxyethyl phosphorylcholine, preferably methacryloyloxyethyl phosphorylcholine.
[3] The single-stranded nucleic acid denaturant of the aforementioned [1] or [2], wherein the monomer having a carboxy group is at least one selected from the group consisting of (meth)acrylic acid and crotonic acid, preferably (meth)acrylic acid, more preferably methacrylic acid.
[4] The single-stranded nucleic acid denaturant of any one of the aforementioned [1] to [3], wherein the content of constitutional units (a) relative to the total constitutional units of the copolymer is 10 to 80 mol%, preferably 20 to 70 mol%, more preferably 25 to 40 mol%.
[5] The single-stranded nucleic acid denaturant of any one of the aforementioned [1] to [4], wherein the content of constitutional units (b) relative to the total constitutional units of the copolymer is 30 to 80 mol%, preferably 60 to 75 mol%.
[6] The single-stranded nucleic acid denaturant of any one of the aforementioned [1] to [5], wherein the copolymer optionally contains constitutional units (c) derived from other monomer different from a monomer having a phosphorylcholine group and a monomer having a carboxy group, and the content of constitutional units (c) relative to the total constitutional units of the copolymer is 0 to 10 mol%, preferably 0 to 5 mol%.
[7] The single-stranded nucleic acid denaturant of any one of the aforementioned [1] to [5], wherein the copolymer does not contain constitutional units (c) derived from other monomer different from a monomer having a phosphorylcholine group and a monomer having a carboxy group.
[8] The single-stranded nucleic acid denaturant of any one of the aforementioned [1] to [7], wherein the copolymer has a weight average molecular weight of 10,000 to 2,000,000, preferably 50,000 to 500,000, more preferably 100,000 to 500,000, further preferably 200,000 to 500,000.
[9] The single-stranded nucleic acid denaturant of any one of the aforementioned [1] to [8], wherein the single-stranded nucleic acid is RNA.
[10] A nucleic acid aqueous solution comprising the single-stranded nucleic acid denaturant of any one of the aforementioned [1] to [8] and a single-stranded nucleic acid.
[11] The nucleic acid aqueous solution of the aforementioned [10], wherein the single-stranded nucleic acid is RNA.
[12] A nucleic acid amplification method using the nucleic acid aqueous solution of the aforementioned [10] or [11].
[13] The nucleic acid amplification method of the aforementioned [12], in which an enzyme reaction is performed.
[14] A method for electrophoresis of a single-stranded nucleic acid, comprising applying the nucleic acid aqueous solution of the aforementioned [10] or [11] to an electrophoresis gel.
[15] A method for denaturing a single-stranded nucleic acid, comprising mixing the single-stranded nucleic acid denaturant of any one of the aforementioned [1] to [8], a single-stranded nucleic acid, and water.
[16] The method of the aforementioned [15], wherein the single-stranded nucleic acid is RNA.

### [Advantageous Effects of Invention]

Using the single-stranded nucleic acid denaturant of the present invention in the separation of single-stranded nucleic acid by electrophoresis method, good separation of the nucleic acid can be achieved by electrophoresis, even under non-denaturing conditions in which no denaturant is added to the electrophoresis buffer or gel, and even without boiling the sample prior to electrophoresis. In addition, since the single-stranded nucleic acid denaturant of the present invention is a synthetic polymer, it is easy to handle as an aqueous solution.

Moreover, since the single-stranded nucleic acid denaturant of the present invention does not inhibit enzyme reactions, a nucleic acid solution containing the aforementioned denaturant and a single-stranded nucleic acid can be used directly in an enzyme reaction. Therefore, as a secondary effect of the single-stranded nucleic acid denaturation effect, the efficiency of the reaction is expected to be improved by loosening the higher-order structure of the nucleic acid, in enzyme reactions using single-stranded nucleic acids as substrates. When the nucleic acid is used as a probe or guide RNA, the specificity of hybridization is expected to be improved by loosening the higher-order structure of the nucleic acid.

### [Brief Description of Drawings]

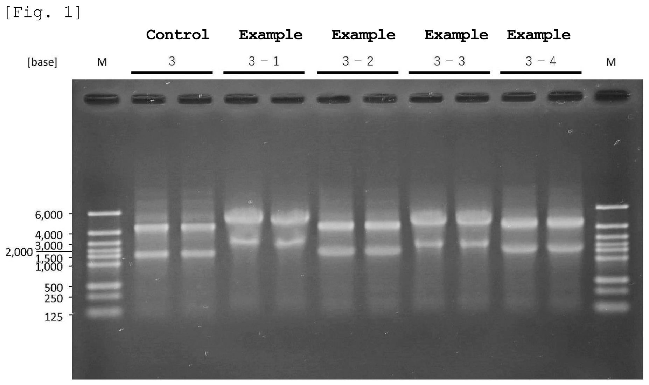

[Fig. 1] Fig. 1 is a photograph showing the results of electrophoresis in Experimental Examples 1 to 3.

### [Description of Embodiments]

The present invention is described in detail below. Respective descriptions in the present specification can be combined with each other, unless it is clear that they cannot be combined.

When stepwise numerical ranges are described in the present specification, the lower limit and the upper limit of each numerical range can be combined. In the case of, for example, "preferably 10 to 100, more preferably 20 to 90", the "preferred lower limit 10" can be combined with the "more preferred upper limit 90" (i.e., the numerical range of "10 to 90" is also within the range of the present specification).

In the present specification, the "single-stranded nucleic acid" means "RNA or single-stranded DNA". The single-stranded nucleic acid is preferably RNA.

In the present specification, the "single-stranded nucleic acid denaturant" means "an additive used to loosen the higher-order structure of single-stranded nucleic acid".

In the present specification, the "denaturation of single-stranded nucleic acid" means "loosening the higher-order structure of single-stranded nucleic acid".

In the present specification, the "constitutional units" means repeat units derived from monomers in polymers. Therefore, the "constitutional units" do not include structures not repeated in the polymer (e.g., structure derived from polymerization initiator and the like).

In the present specification, the "phosphorylcholine group" means a monovalent group represented by the following formula (in the following formula, * indicates a bonding position).

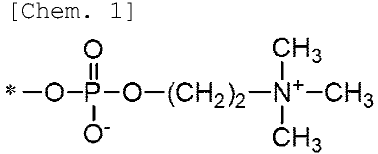

In the present specification, the "(meth)acrylic acid" basically means an "acrylic acid or methacrylic acid". When a plurality of the (meth)acrylic acids may be present, the "(meth)acrylic acid" means an "acrylic acid and/or methacrylic acid". Other terms similar to the "(meth)acrylic acid" also mean the same as the "(meth)acrylic acid".

### [Single-stranded nucleic acid denaturant]

The single-stranded nucleic acid denaturant of the present invention (hereinafter to be referred to as "the denaturant of the present invention") is a copolymer containing constitutional units (a) derived from a monomer having a phosphorylcholine group (hereinafter to be referred to as "monomer a") and constitutional units (b) derived from a monomer having a carboxy group (hereinafter to be referred to as "monomer b"). In the present specification, a monomer having a phosphorylcholine group and a carboxy group is classified as "monomer a".

Examples of the monomer a include (meth)acryloyloxyethyl phosphorylcholine. Only one kind of monomer a may be used, or two kinds thereof may be used in combination. As monomer a, methacryloyloxyethyl phosphorylcholine is preferred from the aspect of the preservation stability of the denaturant of the present invention (i.e., the aforementioned copolymer).

Examples of the monomer b include (meth)acrylic acid, crotonic acid, 3-methylcrotonic acid, angelic acid, tiglic acid, fumaric acid, maleic acid, itaconic acid, citraconic acid and the like. Only one kind of monomer b may be used, or two or more kinds thereof may be used in combination. As monomer b, (meth)acrylic acid or crotonic acid is preferred, (meth)acrylic acid is more preferred, and methacrylic acid is further preferred, from the aspect of the ease of polymerization reactions.

The denaturant of the present invention may contain, as long as the effect of the present invention is not impaired, constitutional units (c) derived from other monomers different from monomer a and monomer b. Examples of other monomer include alkyl (meth)acrylate (the carbon number of the alkyl group is preferably 1 to 6, more preferably 4 to 6), glycerol mono(meth)acrylate, polyethylene glycol (meth)acrylate, and the like. Only one kind of other monomer may be used, or two or more kinds thereof may be used in combination.

In order to not inhibit the electrophoresis of single-stranded nucleic acids, it is preferable that none of the constitutional units (a) to (c) contain cationic functional groups, and it is also preferable that the denaturant of the present invention does not contain a cationic functional group. As used herein, the "cationic functional group" means a functional group that carries a positive charge in aqueous solution, and does not include amphoteric functional groups such as phosphorylcholine groups that carry both positive charge and negative charge and are electrically neutral.

The content of constitutional units (a) relative to the total constitutional units of copolymer is preferably 10 to 80 mol%, more preferably 20 to 70 mol%, further preferably 25 to 40 mol%.

The content of constitutional units (b) relative to the total constitutional units of the copolymer is preferably 20 to 90 mol%, more preferably 30 to 80 mol%, further preferably 60 to 75 mol%, from the aspect of denaturation effect.

The content of constitutional units (c) relative to the total constitutional units of the copolymer is preferably 0 to 10 mol%, more preferably 0 to 5 mol%, from the aspect of denaturation effect. The denaturant of the present invention more preferably does not contain a constitutional unit (c) from the aspect of denaturation effect.

The denaturant of the present invention may be a random copolymer, an alternating copolymer, a block copolymer, a graft polymer, or a copolymer containing two or more structures thereof. It is preferably a random copolymer from the aspect of the manufacturability.

As each monomer for the preparation of the denaturant of the present invention, a commercially available product may be used, or the monomer may be produced by a known method. The denaturant of the present invention can be produced by known methods (e.g., the method described in WO 2018/216628, etc.).

The weight average molecular weight of the denaturant of the present invention is not particularly limited. It is preferably 10,000 to 2,000,000, more preferably 50,000 to 500,000, further preferably 100,000 to 500,000, particularly preferably 200,000 to 500,000, from the aspect of denaturation effect. The weight average molecular weight can be calculated based on polyethylene glycol by gel filtration chromatography measurement (hereinafter sometimes to be abbreviated as "GPC").

Only one kind of the denaturant of the present invention may be used, or two or more kinds thereof may be used in combination. In addition, the denaturant of the present invention may be used in combination with other components (e.g., other denaturant).

### [Nucleic acid aqueous solution]

The present invention further provides a nucleic acid aqueous solution containing the denaturant of the present invention and a single-stranded nucleic acid.

The concentration of the denaturant of the present invention in a nucleic acid aqueous solution is preferably 0.01 to 1.0w/v%, more preferably 0.02 to 0.5w/v%, further preferably 0.05 to 0.2w/v%, from the aspects of denaturation effect and separation of single-stranded nucleic acid (particularly RNA) by length.

Only one kind of single-stranded nucleic acid may be used, or two or more kinds thereof may be used in combination. The nucleic acid aqueous solution of the present invention preferably contains one kind of single-stranded nucleic acid, more preferably one kind of RNA.

The single-stranded nucleic acid may be synthesized, for example, in vitro or by PCR, or may be provided as a virus, bacterial cell, cell, body fluid, tissue, etc., or a suspension of any of these, or a nucleic acid extract, etc., prepared from any of these. The virus, bacterial cell, cell, body fluid, tissue, etc. may be collected from human, animals, or plants in the natural world or environment, or may be obtained by isolation and culture. The concentration of single-stranded nucleic acid in the nucleic acid aqueous solution is determined appropriately according to the application using the nucleic acid.

The nucleic acid aqueous solution of the present invention may further contain other components different from the denaturant of the present invention and single-stranded nucleic acid, as long as the effects of the present invention are not impaired. Examples of other component include buffer, nucleic acid staining reagent, colorant and the like. Only one kind of other component may be used, or two or more kinds thereof may be used in combination.

The buffer is not particularly limited, and examples include Good's buffer, glycine buffer, Tris buffer, phosphate buffer, citrate buffer, and the like. The buffer may further contain a salt such as magnesium salt, calcium salt, manganese salt, potassium chloride, ammonium sulfate, and the like. The buffer may further contain a water-soluble organic solvent such as dimethyl sulfoxide, dimethylformamide, formamide, glycerol, and the like. The buffer may further contain a surfactant such as polyoxysorbitan fatty acid ester, polyoxyethylene alkylphenyl ether, and the like. The buffer may further contain a protein such as bovine serum albumin and the like. The buffer may further contain a water-soluble polymer such as polyethylene glycol and the like. In addition, the buffer may further contain enzymes such as DNA polymerase, nuclease, and protease, as well as their substrates and coenzymes.

Examples of the nucleic acid staining reagent include ethidium bromide, SYBR^{™} Green, and the like.

Examples of the colorant include Orange G, Bromophenol Blue, Xylene Cyanol FF, and the like.

### [Nucleic acid amplification method]

The present invention also provides a nucleic acid amplification method using the nucleic acid aqueous solution of the present invention. Since the denaturant of the present invention does not inhibit enzyme reactions, the nucleic acid amplification method of the present invention is preferably a nucleic acid amplification method that involves an enzyme reaction.

Examples of the enzyme reaction include cleavage by nuclease, reverse transcription by reverse transcriptase, replication by DNA polymerase, and the like. The nucleic acid amplification method of the present invention is preferably PCR (Polymerase Chain Reaction), more preferably reverse transcription PCR.

### [Electrophoresis method of single-stranded nucleic acid]

The present invention also provides an electrophoresis method including applying the nucleic acid aqueous solution of the present invention to an electrophoresis gel. The conditions for electrophoresis method are well known to those skilled in the art. Therefore, those skilled in the art can appropriately set the conditions to perform electrophoresis method. Furthermore, electrophoresis gels are also well known to those skilled in the art. Therefore, no particular limitation is imposed on the electrophoresis gel in the present invention.

### [Method for denaturating single-stranded nucleic acid]

The present invention also provides a method for denaturing single-stranded nucleic acids, including mixing the single-stranded nucleic acid denaturant of the present invention, a single-stranded nucleic acid, and water. The aforementioned mixing is not particularly limited. For example, (1) the single-stranded nucleic acid denaturant of the present invention, a single-stranded nucleic acid, and water may be mixed; (2) an aqueous solution of a single-stranded nucleic acid and the single-stranded nucleic acid denaturant of the present invention may be mixed; or (3) an aqueous solution of the single-stranded nucleic acid denaturant of the present invention and the single-stranded nucleic acid denaturant may be mixed. The explanations of the nucleic acid aqueous solution obtained by mixing the single-stranded nucleic acid denaturant of the present invention, a single-stranded nucleic acid, and water (e.g., the kind of nucleic acid used, the concentration of the single-stranded nucleic acid denaturant of the present invention, and other components) are the same as those of the aforementioned nucleic acid aqueous solution of the present invention.

### [Example]

The present invention is explained in more detail in the following by referring to the following Synthetic Examples and the like, which are not to be construed as limitative.

### [Synthesis of polymer]

Copolymer 1-1 to copolymer 1-7, homopolymer 2, and copolymer 3 and copolymer 4 were prepared as follows. Copolymer 1-1 to copolymer 1-5 are denaturants of the present invention, and copolymer 1-6, copolymer 1-7, homopolymer 2, copolymer 3 and copolymer 4 are single-stranded nucleic acid denaturants outside the scope of the present invention (hereinafter to be referred to as "the denaturant of Comparative Example").

The obtained polymers were dissolved in "Water, Nuclease-free" (hereinafter referred to as "PW") manufactured by NIPPON GENE CO., LTD. to a concentration 10 times the final concentration described under each condition in the below-mentioned Examples and Comparative Examples, and the obtained polymer aqueous solutions were used.

### [Synthetic Example 1-1]

2-Methacryloyloxyethyl phosphorylcholine (hereinafter to be indicated as "MPC") (60 g) and methacrylic acid (hereinafter indicated as "MAc") (40 g) (MPC/MAc=30/70 (molar ratio)) were weighed into a glass flask for polymerization and 567 g of purified water was added to dissolve them. To the obtained solution was added 4,4'-azobis(4-cyanovaleric acid) (hereinafter indicated as "ACVA") (5.2 g). The solution was heated to 70°C and stirred under a nitrogen atmosphere for 6 hr to perform polymerization. The obtained polymerization solution was purified by dialysis using a semipermeable membrane with a molecular cutoff of 20,000, and then freeze-dried to obtain copolymer 1-1. The weight average molecular weight of copolymer 1-1 was 370,000 based on polyethylene glycol as measured by GPC under the below-mentioned conditions.

### [Synthetic Example 1-2]

MPC (60 g) and MAc (40 g) (MPC/MAc=30/70 (molar ratio)) were weighed into a glass flask for polymerization and 1,000 g of purified water was added to dissolve them. To the obtained solution was added ACVA (77.3 g). The solution was heated to 70°C and stirred under a nitrogen atmosphere for 6 hr to perform polymerization. The obtained polymerization solution was purified by dialysis using a semipermeable membrane with a molecular cutoff of 3,000, and then freeze-dried to obtain copolymer 1-2. The weight average molecular weight of copolymer 1-2 was 40,000 based on polyethylene glycol as measured by GPC under the below-mentioned conditions.

### [Synthetic Example 1-3]

MPC (77 g) and MAc (23 g) (MPC/MAc=50/50 (molar ratio)) were weighed into a glass flask for polymerization and 400 g of purified water was added to dissolve them. To the obtained solution was added ACVA (3.9 g). The solution was heated to 70°C and stirred under a nitrogen atmosphere for 6 hr to perform polymerization. The obtained polymerization solution was purified by dialysis using a semipermeable membrane with a molecular cutoff of 20,000, and then freeze-dried to obtain copolymer 1-3. The weight average molecular weight of copolymer 1-3 was 260,000 based on polyethylene glycol as measured by GPC under the below-mentioned conditions.

### [Synthetic Example 1-4]

MPC (77 g) and MAc (23 g) (MPC/MAc=50/50 (molar ratio)) were weighed into a glass flask for polymerization and 400 g of purified water was added to dissolve them. To the obtained solution was added ACVA (7.8 g). The solution was heated to 70°C and stirred under a nitrogen atmosphere for 6 hr to perform polymerization. The obtained polymerization solution was purified by dialysis using a semipermeable membrane with a molecular cutoff of 3,000, and then freeze-dried to obtain copolymer 1-4. The weight average molecular weight of copolymer 1-4 was 70,000 based on polyethylene glycol as measured by GPC under the below-mentioned conditions.

### [Synthetic Example 1-5]

MPC (89 g) and MAc (11 g) (MPC/MAc=70/30 (molar ratio)) were weighed into a glass flask for polymerization and 400 g of purified water was added to dissolve them. To the obtained solution was added ACVA 3.3g. The solution was heated to 70°C and stirred under a nitrogen atmosphere for 6 hr to perform polymerization. The obtained polymerization solution was purified by dialysis using a semipermeable membrane with a molecular cutoff of 20,000, and then freeze-dried to obtain copolymer 1-5. The weight average molecular weight of copolymer 1-5 was 250,000 based on polyethylene glycol as measured by GPC under the below-mentioned conditions.

### [Synthetic Example 1-6]

MPC (97 g) and MAc (3 g) (MPC/MAc=90/10 (molar ratio)) were weighed into a glass flask for polymerization and 400 g of purified water was added to dissolve them. To the obtained solution was added ACVA (3.3 g). The solution was heated to 70°C and stirred under a nitrogen atmosphere for 6 hr to perform polymerization. The obtained polymerization solution was purified by dialysis using a semipermeable membrane with a molecular cutoff of 20,000, and then freeze-dried to obtain copolymer 1-6. The weight average molecular weight of copolymer 1-6 was 210,000 based on polyethylene glycol as measured by GPC under the below-mentioned conditions.

### [Synthetic Example 1-7]

MPC (99 g) and MAc (1 g) (MPC/MAc=95/5 (molar ratio)) were weighed into a glass flask for polymerization and 400 g of purified water was added to dissolve them. To the obtained solution was added ACVA (3.3 g). The solution was heated to 70°C and stirred under a nitrogen atmosphere for 6 hr to perform polymerization. The obtained polymerization solution was purified by dialysis using a semipermeable membrane with a molecular cutoff of 20,000, and then freeze-dried to obtain copolymer 1-7. The weight average molecular weight of copolymer 1-7 was 100,000 based on polyethylene glycol as measured by GPC under the below-mentioned conditions.

### [Synthetic Example 2]

MPC (100 g) was weighed into a glass flask for polymerization and 150 g of purified water was added to dissolve them. To the obtained solution was added ACVA (2.0 g). The solution was heated to 70°C and stirred under a nitrogen atmosphere for 6 hr to perform polymerization. The obtained polymerization solution was purified by dialysis using a semipermeable membrane with a molecular cutoff of 20,000, and then freeze-dried to obtain homopolymer 2. The weight average molecular weight of homopolymer 2 was 1,030,000 based on polyethylene glycol as measured by GPC under the below-mentioned conditions.

### [Synthetic Example 3]

MPC (74 g) and N,N,N-trimethyl-N-(2-hydroxy-3-methacryloyloxypropyl)-ammonium chloride (hereinafter indicated as "QA") (26 g) (MPC/QA=70/30 (molar ratio)) were weighed into a glass flask for polymerization and 525 g of purified water was added to dissolve them. To the obtained solution was added 2,2'-azobis(2-methylpropionamide)dihydrochloride (0.9 g). The solution was heated to 70°C and stirred under a nitrogen atmosphere for 2 hr to perform polymerization. The obtained polymerization solution was purified by dialysis using a semipermeable membrane with a molecular cutoff of 20,000, and then freeze-dried to obtain copolymer 3. The weight average molecular weight of copolymer 3 was 40,000 based on polyethylene glycol as measured by GPC under the below-mentioned conditions.

### [Synthetic Example 4]

MPC (57 g) and butyl methacrylate (hereinafter indicated as "BMA") (28 g), glycerol monomethacrylate (hereinafter indicated as "GLM") (15 g) (MPC/BMA/GLM=40/40/20 (molar ratio)) were weighed into a glass flask for polymerization and ethanol (409 g) and purified water (201 g) were added to dissolve them. To the obtained solution was added 2,2'-azobis(isobutyronitrile) (1.9 g). The solution was heated to 60°C and stirred under a nitrogen atmosphere for 5 hr to perform polymerization. The obtained polymerization solution was purified by dialysis using a semipermeable membrane with a molecular cutoff of 3,000, and then freeze-dried to obtain copolymer 4. The weight average molecular weight of copolymer 4 was 20,000 based on polyethylene glycol as measured by GPC under the below-mentioned conditions.

### [GPC measurement]

GPC measurement of the obtained polymers was performed under the following conditions.
GPC system: EcoSEC system (manufactured by Tosoh Corporation)
column: Shodex OHpak SB-802.5HQ (manufactured by Showa Denko K.K.), and SB-806M HQ (manufactured by Showa Denko K.K.) connected in tandem
eluent: 20 mM sodium phosphate buffer (pH 7.4)
detector: differential refractive index detector
molecular weight standard: EasiVial PEG/PEO (manufactured by Agilent Technologies)
flow rate: 0.5 mL/min
column temperature: 40°C
sample: obtained polymer diluted with eluent to final concentration of 0.1 wt%
injection volume: 100 µL

The kinds and contents of the monomers used in the synthesis of the polymers (i.e., the content of the constitutional unit derived from the monomer), and the weight average molecular weights of the obtained polymers are described in Table 1.

**[Table 1]**

| | monomer (a) | | monomer (b) | | other monomer | | weight average molecular weight [×10³] |
|---|---|---|---|---|---|---|---|
| | kind | content [mol%] | kind | content [mol%] | kind | content [mol%] | |
| copolymer 1-1 | MPC | 30 | MAc | 70 | - | - | 370 |
| copolymer 1-2 | MPC | 30 | MAc | 70 | - | - | 40 |
| copolymer 1-3 | MPC | 50 | MAc | 50 | - | - | 260 |
| copolymer 1-4 | MPC | 50 | MAc | 50 | - | - | 70 |
| copolymer 1-5 | MPC | 70 | MAc | 30 | - | - | 250 |
| copolymer 1-6 | MPC | 90 | MAc | 10 | - | - | 210 |
| copolymer 1-7 | MPC | 95 | MAc | 5 | - | - | 100 |
| homopolymer 2 | MPC | 100 | - | - | - | - | 1030 |
| copolymer 3 | MPC | 70 | - | - | QA | 30 | 40 |
| copolymer 4 | MPC | 40 | - | - | BMA | 40 | 20 |
| | | | | | GLM | 20 | |

MPC: 2-methacryloyloxyethyl phosphorylcholine
MAc: methacrylic acid
QA: N,N,N-trimethyl-N-(2-hydroxy-3-methacryloyloxypropyl)-ammonium chloride
BMA: butyl methacrylate
GLM: glycerol monomethacrylate

### [Experimental Example 1]

The denaturant of the present invention, the denaturant of Comparative Example, or the conventional denaturant described below was added to an RNA solution to prepare samples, and agarose gel electrophoresis was performed. In this case, the gel and buffer used for electrophoresis were those under non-denaturing conditions (i.e., no denaturant was added).

### [Common Procedures]

1) As the total RNA used in Experimental Example 1-1 to Experimental Example 1-3, Human HeLa Cell Total RNA (Clontech) was added by 1 µg per experiment.
2) As the 10X buffer used in Experimental Example 1-1 to Experimental Example 1-3, 400 mM Tris-HCl buffer (containing 100 mM sodium chloride, 60 mM magnesium chloride, 10 mM calcium chloride, pH 7.9) was prepared.
3) As the electrophoresis buffer, 50X TAE (manufactured by NIPPON GENE CO., LTD.) diluted (1X TAE) according to the manufacturer's instructions was used.
4) As the electrophoresis gel, 100 mL of 1X TAE was added to Agarose S (manufactured by NIPPON GENE CO., LTD.) (1 g), boiled to dissolve in a microwave oven, solidified, and used.
5) For electrophoresis, 1/6-fold amount of 6X Loading Buffer, Triple Dye (manufactured by NIPPON GENE CO., LTD.) was added to each sample and mixed, and 5 µL of this mixture was applied to the electrophoresis gel, and electrophoresis was performed at 100V for about 30 min.
6) Electrophoresis images were taken by staining the sample by immersing same for 15 min in Midori Green Advance (manufactured by Nippon Genetics Co., Ltd.) diluted 10,000-fold with 1X TAE, then bleaching same by immersion in pure water for 15 min, and then taking photographs under BlueGreen LED illumination.

### [Experimental Example 1-1]

1) Six types of RNA solutions shown in Table 2 were prepared. Example 1 alone is within the range of the present invention.
2) Immediately (without boiling), agarose gel electrophoresis was performed and developed using the procedure described above.
3) The electrophoretic image was confirmed, and the positions of the two rRNA bands (theoretical values: 5.0 kbase and 1.9 kbase) were compared with the control. Those that showed a shift toward higher molecular weights were judged to have an RNA denaturation effect (O), and those that did not were judged to have no RNA denaturation effect (×). The results are shown in Table 2.

**[Table 2]**

| | | | control 1 | Example 1 | Comparative Example 1-1 | Comparative Example 1-2 | Comparative Example 1-3 | Comparative Example 1-4 |
|---|---|---|---|---|---|---|---|---|
| composition | 10X buffer | | 2 µL | | | | | |
| | Total RNA | | 1 µL (total RNA amount: 1 µg) | | | | | |
| | polymer aqueous solution | kind of polymer | - | copolymer 1-1 | homopolymer 2 | copolymer 3 | copolymer 4 | - |
| | | amount added (*1) | - | 2 µL | 2 µL | 2 µL | 2 µL | - |
| | other | kind | - | - | - | - | - | FAm (*2) |
| | | amount added (*3) | - | - | - | - | - | 2 µL |
| | PW | | rest | | | | | |
| | total amount | | 20 µL | | | | | |
| results | RNA denaturation effect | | - | ○ | × | × | × | × |

| | | | | | | | | |
|---|---|---|---|---|---|---|---|---|
| (*1) polymer final concentration: 0.5 w/v% (*2) Fam: formamide (manufactured by FUJIFILM Wako Pure Chemical Corporation) (*3) Fam final concentration: 10 v/v% | | | | | | | | |

From the results shown in Table 2, it has been found that the denaturant of the present invention (copolymer 1-1) can preferably denature RNA, which is a single-stranded nucleic acid, even when the gel and buffer for electrophoresis are under non-denaturing conditions and the electrophoresis sample is not boiled.

Comparative Example 1-1 is an experimental example in which homopolymer 2 composed of monomers a was used. Since RNA denaturation was not achieved in Comparative Example 1-1, it is clear that the constitutional unit (b) is necessary for the RNA denaturation effect.

Comparative Example 1-2 is an experimental example in which copolymer 3 was used, instead of constitutional units (b), which contained constitutional units (c) derived from monomer (QA) having a cationic functional group, and Comparative Example 1-3 is an experimental example in which copolymer 4 was used, instead of constitutional units (b), which contained constitutional units (c) derived from monomers (BMA and GLM) having no carboxy group. Since RNA denaturation was not achieved in Comparative Example 1-2 and Comparative Example 1-3, it is clear that the constitutional unit (b) having a carboxy group is necessary for the RNA denaturation effect.

Comparative Example 1-4 is an experimental example in which a general-purpose denaturant formamide was used. RNA denaturation was not achieved in Comparative Example 1-4. This is considered to be because the denaturation effect of formamide is weak, and a sufficient denaturation effect is not achieved when the gel and buffer for electrophoresis are used under non-denaturation conditions and the electrophoresis sample is not boiled.

### [Experimental Example 1-2]

1) Seven types of RNA solutions shown in Table 3 were prepared.
2) From thereon, the same procedures and evaluations as in Experimental Example 1-1 were performed. The results are shown in Table 3.

**[Table 3]**

| | | | control 2 | Example 2-1 | Example 2-2 | Example 2-3 | Comparative Example 2-1 | Comparative Example 2-2 | Comparative Example 2-3 |
|---|---|---|---|---|---|---|---|---|---|
| composition | 10X buffer | | 2 µL | | | | | | |
| | Total RNA | | 1 µL (total RNA amount: 1 µg) | | | | | | |
| | polymer aqueous solution | kind of polymer | | copolymer 1-1 | copolymer 1-3 | copolymer 1-5 | copolymer 1-6 | copolymer 1-7 | |
| | | amount added (*1) | - | 2 µL | 2 µL | 2 µL | 2 µL | 2 µL | - |
| | other | kind | - | - | - | - | - | - | PMAc (*2) |
| | | amount added (*3) | - | - | - | - | - | - | 2 µL |
| | PW | | rest | | | | | | |
| | total amount | | 20 µL | | | | | | |
| results | RNA denaturation effect | | - | ○ | ○ | ○ | × | × | ○ |

| | | | | | | | | | |
|---|---|---|---|---|---|---|---|---|---|
| (*1) polymer final concentration: 0.5 w/v% (*2) PMAc: polymethacrylic acid (manufactured by Sigma-Aldrich) (*3) PMAc final concentration: 0.5 w/v% | | | | | | | | | |

Example 2-1 to Example 2-3 are experimental examples using the denaturants of the present invention (copolymer 1-1, copolymer 1-3, or copolymer 1-5), Comparative Example 2-1 and Comparative Example 2-2 are experimental examples using the denaturants of Comparative Example (copolymer 1-6 or copolymer 1-7) whose content of constitutional units (b) is outside the range of the present invention. From these comparisons, it is clear that the content of constitutional units (b) needs to be adjusted to an appropriate range in order to achieve the RNA denaturation effect.

On the other hand, Comparative Example 2-3 is an experimental example in which polymethacrylic acid was used instead of the denaturant of the present invention. In Comparative Example 2-3, RNA denaturation effect was obtained. Polymethacrylic acid was further examined in Experimental Example 2 described below.

### [Experimental Example 1-3]

1) Five types of RNA solutions shown in Table 4 were prepared.
2) From thereon, the same procedures and evaluations as in Experimental Example 1-1 were performed. The results are shown in Table 4 and Fig. 1.

M in Fig. 1 indicates the results obtained using molecular weight markers. To be specific, M indicates the results when a mixture of 5 µL of PW, 1.5 µL of 10X TE (manufactured by DOJINDO LABORATORIES), 1 µL of RNA Ladder (manufactured by NIPPON GENE CO., LTD.), and 7.5 µL of 2X RNA loading buffer (ethidium bromide-free) (manufactured by FUJIFILM Wako Pure Chemical Corporation) was heated in a 70°C water bath for 10 min, immediately cooled on ice, and 5 µL thereof was applied.

The numbers on the left side of Fig. 1 indicate the RNA length [base] corresponding to the molecular weight marker. Human 28S rRNA is 5.0 kbase, and human 18S rRNA is 1.9 kbase.

**[Table 4]**

| | | | control 3 | Example 3-1 | Example 3-2 | Example 3-3 | Example 3-4 |
|---|---|---|---|---|---|---|---|
| composition | 10X buffer | | 2 µL | | | | |
| | Total RNA | | 1 µL (total RNA amount: 1 µg) | | | | |
| | polymer aqueous solution | kind of polymer | - | copolymer 1-1 | copolymer 1-2 | copolymer 1-3 | copolymer 1-4 |
| | | amount added (*1) | - | 2 µL | 2 µL | 2 µL | 2 µL |
| | PW | | rest | | | | |
| | total amount | | 20 µL | | | | |
| results | RNA denaturation effect | | - | ○ | ○ | ○ | ○ |

| | | | | | | | |
|---|---|---|---|---|---|---|---|
| (*1) polymer final concentration: 0.5 w/v% | | | | | | | |

Example 3-1 to Example 3-4 are experimental examples using the denaturants of the present invention (copolymer 1-1 to copolymer 1-4), and as shown in Table 4, all of them exhibited an RNA denaturation effect. However, as shown in Fig. 1, when Example 3-1 using copolymer 1-1 is compared with Example 3-2 using copolymer 1-2, the degree of shifting of the two rRNA bands to a higher molecular weight side is more pronounced in Example 3-1 using copolymer 1-1 with a higher molecular weight. Furthermore, when Example 3-3 using copolymer 1-3 is compared with Example 3-4 using copolymer 1-4, the degree of shifting of the two rRNA bands to a higher molecular weight side is more pronounced in Example 3-3 using copolymer 1-3 with a higher molecular weight. This shows that when the kind and content of the constitutional units are the same, the denaturants of the present invention with a higher weight average molecular weight exhibit a superior RNA denaturation effect compared to the denaturants of the present invention with a lower weight average molecular weight.

### [Experimental Example 2]

The influence of the single-stranded nucleic acid denaturant on the enzyme reaction was examined. An RNA solution containing the denaturant of the present invention was subjected to reverse transcription PCR using an enzyme reaction.
1) Three types of RNA solutions shown in Table 5 were prepared.
2) The reverse transcription PCR solution shown in Table 6 was prepared and dispensed into a PCR plate (MicroAmp^{™} Fast Optical 96-well Reaction Plate with Barcode, 0.1 mL, manufactured by Applied Bioscience).
3) The plate was set in a StepOnePlus^{™} Real-Time PCR System (manufactured by Applied Bioscience), and reverse transcription PCR was performed using the temperature profile shown in Table 7.
4) The fluorescence intensity of FAM derived from probe 1-P and the fluorescence intensity of the passive reference (50 x ROX Passive Reference, manufactured by NIPPON GENE CO., LTD.) were read, and an amplification curve was output, plotting the cycle number on the horizontal axis and ΔRn on the vertical axis.
5) The maximum ΔRn value in the negative control, rounded up to one digit (ΔRn=0.1), was used as the threshold. Experimental Examples in which a ΔRn value exceeding the threshold was output were judged to indicate that the enzyme reaction was not inhibited (○), and Experimental Examples otherwise were judged to indicate that the enzyme reaction was inhibited (×). The results are shown in Table 5.

**[Table 5]**

| | | | control 4 | Example 4 | Comparative Example 4 |
|---|---|---|---|---|---|
| composition | Control RNA, N2 gene (*1) | | 2 µL | | |
| | polymer aqueous solution | kind of polymer | - | copolymer 1-1 | - |
| | | amount added (*2) | - | 2 µL | - |
| | other | kind | - | - | PMAC (*3) |
| | | amount added (*4) | - | - | 2 µL |
| | PW | | rest | | |
| | total amount | | 20 µL | | |
| results | enzyme reaction | | - | ○ | × |

| | | | | | |
|---|---|---|---|---|---|
| (*1) New coronavirus positive control RNA, N2 set, RNA final concentration: 250 copies/uL (*2) polymer final concentration: 0.5 w/v% (*3) PMAc: polymethacrylic acid (manufactured by Sigma-Aldrich) (*4) PMAc final concentration: 0.5 w/v% | | | | | |

**[Table 6]**

| component | liquid volume (µL) |
|---|---|
| 5X buffer for rTth/TTx (*1) | 4 |
| 50 mM Mn(OAc)₂ (*1) | 1 |
| 2 mM dNTPs (*1) | 4 |
| 10 µM Fw Primer NIID_2019-nCOV_N_F2 (*2) | 0.6 |
| 10 µM Rv Primer NIID_2019-nCOV_N_R2 (*2) | 0.6 |
| 10 µM TaqMan Probe NIID_2019-nCOV_N_P2 (*2) | 0.8 |
| Hot Start TTx DNA Polymerase (*1) | 0.3 |
| 50X ROX (*3) | 0.4 |
| RNA solution (*4) | 2 |
| PW | rest |
| total amount | 20 |

| | |
|---|---|
| (*1) constituent component of Hot Start TTx (RNA) Kit (manufactured by TOYOBO) (*2) manufactured by eurofins (*3) manufactured by NIPPON GENE CO., LTD. (*4) RNA solution whose composition is listed in Table 5 | |

**[Table 7]**

| step No. | temperature (°C) | time | cycle number | reading |
|---|---|---|---|---|
| 1 | 90 | 30 sec | - | - |
| 2 | 60 | 5 min | | |
| 3 | 95 | 1 min | | |
| 4 | 95 | 15 sec | 60 | |
| 5 | 60 | 30 sec | | ○ |

Example 4 and Comparative Example 4 are experimental examples using the denaturant (copolymer 1-1 or polymethacrylic acid) that was found to have an RNA denaturation effect in the above-mentioned Experimental Example 1. As shown in Table 5, the enzyme reaction was not inhibited in Example 4, which used copolymer 1-1 containing MPC-derived constitutional units (a) and MAc-derived constitutional units (b), but the enzyme reaction was inhibited in Comparative Example 4, which used polymethacrylic acid consisting of MAc-derived constitutional units (b).

### [Industrial Applicability]

Single-stranded nucleic acid can be favorably denatured by simply adding the denaturant of the present invention to a solution of the single-stranded nucleic acid. Therefore, the denaturant of the present invention can be used to easily separate single-stranded nucleic acids by length in research in the field of molecular biology and various applied researches.

Furthermore, since the denaturant of the present invention does not inhibit enzyme reactions, a nucleic acid solution containing the denaturant of the present invention can be subjected to an enzyme reaction. Therefore, the denaturant of the present invention is expected to be used to improve the reaction efficiency of enzyme reactions using single-stranded nucleic acids as substrates, by utilizing its action of loosening the higher-order structure of single-stranded nucleic acids, and to improve hybridization specificity when using single-stranded nucleic acids as probes or guide RNAs for site-specific nucleases.

This application is based on a patent application No. 2023-055218 filed in Japan, the contents of which are encompassed in full herein.

## Claims

1. A single-stranded nucleic acid denaturant consisting of a copolymer containing constitutional units (a) derived from a monomer having a phosphorylcholine group and constitutional units (b) derived from a monomer having a carboxy group, wherein the content of constitutional units (b) relative to the total constitutional units of the copolymer is 20 to 90 mol%.

2. A nucleic acid aqueous solution comprising the single-stranded nucleic acid denaturant according to claim 1 and a single-stranded nucleic acid.

3. A nucleic acid amplification method using the nucleic acid aqueous solution according to claim 2.

4. The nucleic acid amplification method according to claim 3, in which an enzyme reaction is performed.
